# EUROPEAN PATENT APPLICATION

(11) **EP 2 045 297 A2**
(43) Date of publication of application: **08.04.2009**
(21) Application number: 08253181.5
(22) Date of filing: 30.09.2008
(51) Int. Cl.: C09B 69/00, A61Q 19/04

(54) **Compositions for use in darkening skin**

(30) Priority: 01.10.2007 US 865218
(71) Applicant: Johnson and Johnson Consumer Companies, Inc., Skillman, NJ 08558 (US)
(72) Inventor: Lin, Connie, B., Belle Mead, NJ 08502 (US); Scarpa, Richard, C., Port Washington, NY 10050 (US); Shapiro, Stanley, S., Roseland, NJ 07068 (US)
(74) Representative: Kirsch, Susan Edith

(57) **Abstract**

The present invention features a polymer for darkening the skin. The polymer comprises repeating units derived from melanin precursors and amine-comonomers.
The polymer may be combined with a cosmetically acceptable carriers to form topical compositions. Methods of darkening the skin are also provided.

## Description

### FIELD OF THE INVENTION

The present invention relates to polymers, compositions including said polymers, and the use thereof to darken the skin.

### BACKGROUND OF THE INVENTION

Many individuals desire the darkening of skin color. Most people obtain darker skin through exposure to UV light (e.g., suntanning or UV lamps). UV exposure, however, results in accelerated skin aging and increased incidence of skin cancer. The ability to generate a tanned appearance without incurring photodamage, thus, is important to many individuals. Accordingly, alternative methods for "sunless tanning" have evolved.

One method is the use of products containing dihydroxy acetone (DHA). These products, however, sometimes produce color that is too orange or unnatural to the user. Moreover, the DHA-produced skin color only minimally protects the user from UV irradiation. Products containing beta-carotene, cantaxanthin and lycopene have also been used to darken the skin. These products, however, have no effect at all on melanogenesis and usually result in unnatural and uneven skin color by staining the fat layers just below the skin. In addition, these products do not provide any sun protection as compared to naturally tanned skin.

In order to provide natural appearing tan to skin, so-called "synthetic melanin" derivatives have been devised. Examples of synthetic melanin derivatives are disclosed in U.S. Patent Nos. 5,618,519, 5,384,116, and 5,227,459. Examples of soluble melanin derivatives are disclosed in 5,744,125, 5,225,435, 5,218,079, and 5,216,116. Examples of commercially available soluble melanin derivatives include Melasyn-100™ from San-Mar Laboratories, Inc. (Elmsford, NY) and MelanZe™ from Zylepsis (Ashford, Kent, United Kingdom).

While conventional synthetic melanin derivatives can enhance the body's natural pigment content, enhance photo-protection without the need for UV exposure or the need for medical intervention, and potentially result in a naturally-appearing skin color, these compounds still suffer from certain drawbacks. First, they are made by reacting various monomers together, generally using harsh and highly reactive chemicals. As such, conventional synthetic melanin compounds are relatively unsafe to synthesize. Furthermore, such conventional synthetic melanin synthesis cannot be readily adapted to provide a variety of aesthetic tones and shades to the end-user.

An easy, safe and effective method of making synthetic melanin polymers has now been discovered. Such polymers are obtainable by reacting a melanin precursor with an amine co-monomer under ambient conditions and at low pH. Strong oxidizers are not required. Accordingly, the polymers may even be generated directly on the skin or hair in a safe, fast and inexpensive manner. Incorporation of the polymer into topical compositions yields new sunless tanning products that are safe, easy to use, and provide immediate and natural color.

### SUMMARY OF THE INVENTION

In one aspect, the invention relates to a polymer comprising (i) at least one repeating unit derived from a melanin precursor selected from the group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin and mixtures thereof; and (ii) at least one repeating unit derived from an amine co-monomer having a formula selected from the group consisting of: 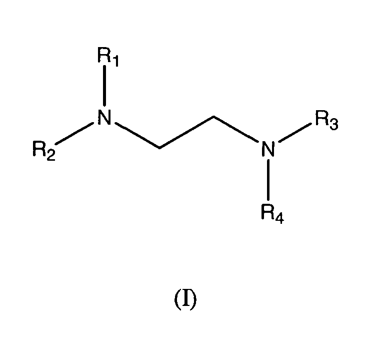 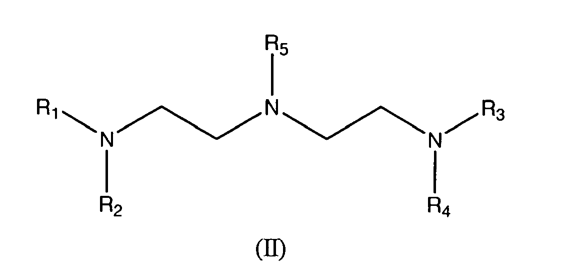 and 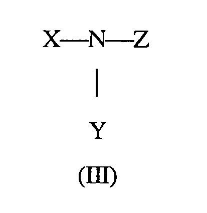 wherein each of R₁, R₂, R₃, R₄, and R₅ independently are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof.

In another aspect of the invention, a topical composition comprising the above polymer and a cosmetically acceptable carrier is provided.

In another aspect of the invention, a method of darkening the skin is provided. The method includes applying the above polymer to the skin.

In another aspect of the invention, a further method of darkening the skin is provided. The method includes applying to the skin a first composition comprising a melanin precursor selected from a group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin, and mixtures thereof; and applying to said skin a second composition comprising an amine co-monomer having a formula selected from the group consisting of: 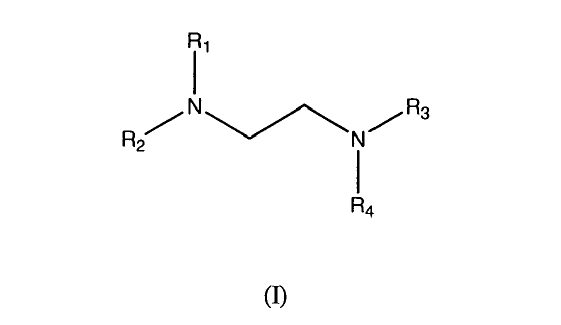 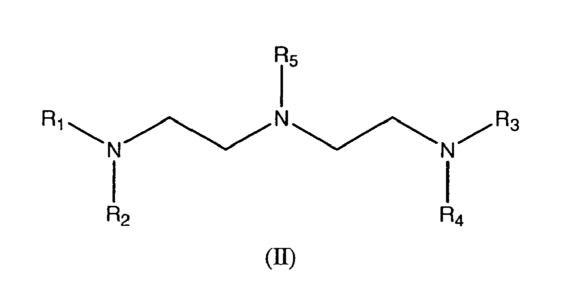 and 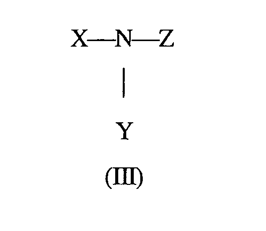 wherein each of R₁, R₂, R₃, R₄, and R₅ are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof. The method further includes allowing said melanin precursor and said amine co-monomer to polymerize on said skin.

In another aspect of the invention, a product is provided. The product comprises a first composition in a first container, wherein said first composition comprises a melanin precursor selected from the group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin, and mixtures thereof. The product further comprises a second composition in a second container separated from said first container, wherein said second composition comprises an amine co-monomer having a formula selected from the group consisting of: 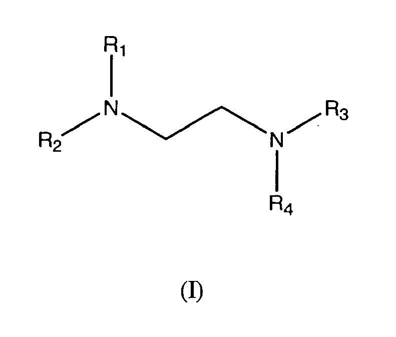 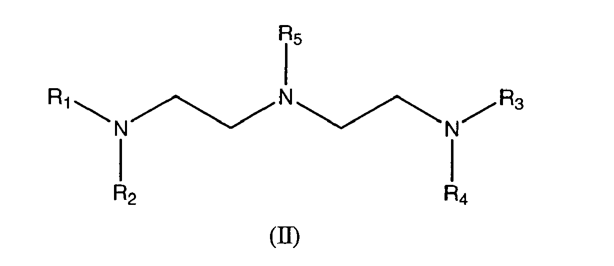 and 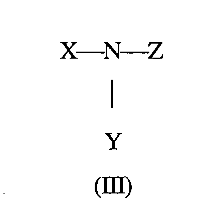 wherein each of R₁, R₂, R₃, R₄, and R₅ are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof.

The invention also provides a polymer obtainable by the reaction of (i) at least one repeating unit derived from a melanin precursor selected from the group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin and mixtures thereof; and (ii) at least one repeating unit derived from an amine co-monomer having a formula selected from the group consisting of: 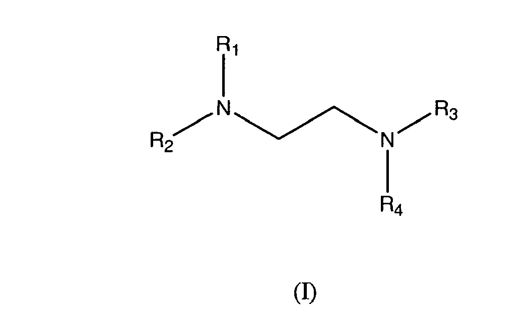 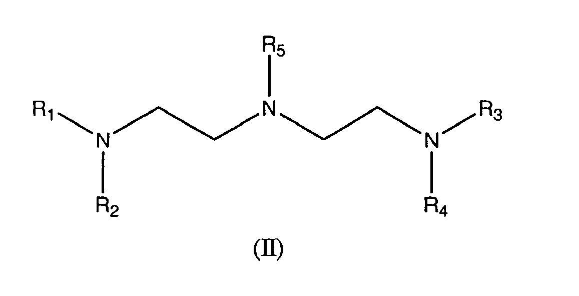 and 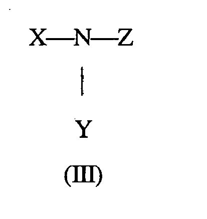 wherein each of R₁, R₂, R₃, R₄, and R₅ independently are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof.

Other features and advantages of the present invention will be apparent from the detailed description of the invention and from the claims.

### DETAILED DESCRIPTION OF THE INVENTION

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

### Definitions

What is meant by "darkening the skin or hair" is darkening the appearance of human skin or hair, including, but not limited to, darkening human skin to either achieve a "sun tan" effect or to cover the light areas of the skin (e.g., as a result of a scar or a disease or a therapy) or darkening natural hair color or restoring discolored hair due to aging (e.g., gray or white hair) or external aggressions (e.g., excess exposure to sun or chlorine).

As used herein, "topically applying" means directly laying on or spreading on outer skin, scalp, or hair, e.g., by use of the hands or an applicator such as a wipe, roller, or spray.

As used herein, "cosmetically acceptable" means that the ingredients described are suitable for use in contact with tissues (e.g., the skin or hair) without undue toxicity, incompatibility, instability, irritation, allergic response, or the like.

As used herein, "safe and effective amount" means an amount sufficient to induce a darkening of human skin or hair, but low enough to avoid serious side effects. The safe and effective amount of the composition will vary with the area being treated, the age and skin type of the end user, the duration and nature of the treatment, the specific ingredient, or composition employed, the particular cosmetically acceptable carrier utilized, and like factors.

### Polymer

Certain embodiments of the invention relate to a polymer useful for darkening the skin. The polymer absorbs UV radiation sufficiently in the visible spectrum to produce a cosmetically acceptable darkening of human skin or hair. The polymer is obtainable by reacting a melanin precursor and an amine co-monomer. The melanin precursor and amine co-monomer may be readily reacted with one another under ambient and mild (mildly alkaline) or low pH conditions that are devoid of strong oxidizers. The polymer is generally readily dispersible or soluble in water or other cosmetically acceptable vehicles. In one embodiment, the polymer has an average molecular weight of from about 10,000 to about 50,000, such as from about 15,000 to about 40,000.

### Melanin Precursor

The polymer comprises at least one repeating unit derived from a melanin precursor. In one embodiment, the melanin precursor includes a hydroxylated aromatic ring with an ionizable side groups. Exemplary melanin precursors include but are not limited to: 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin and mixtures thereof. While the inventors believe that a wide variety of melanin precursors are suitable for making the polymer, particularly suitable melanin precursors are aloin (e.g., C₂₁H₂₂O₉, CAS No. 1415-73-2), dihydroxyphenylalanine (DOPA), vanillin, and combinations thereof. By "aloin" it is meant pure aloin A monomer or alion in aloe extract (∼50%). By "vanillin" it is meant vanillin monomer and/or o-vanillin monomers as described in co-pending, commonly assigned, US Patent Application Publication 20050129633, "Vanillin Polymers For Use In Darkening The Skin," incorporated herein by reference.

### Amine Co-Monomer

The polymer also comprises at least one repeating unit derived from an amine-comonomer. In one embodiment, the amine co-monomer may be a compound of Formula I or II below: 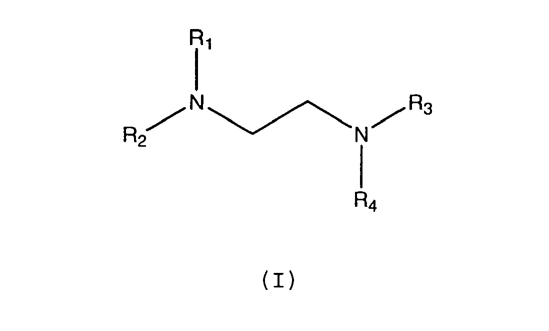 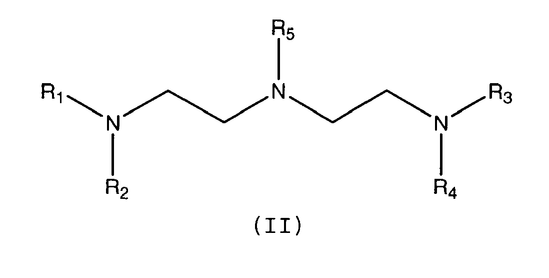 wherein each of R₁, R₂, R₃, R₄, and R₅ are selected from the group consisting of hydrogen, C₁-C₆ alkyl, C₁-C₆ hydroxyalkyl. Examples of suitable amine-comonomers are described in co-pending, commonly assigned, US Patent Application Publication US20060257335, "Compositions Containing Amines and Use Thereof to Darken the Skin."

In one embodiment, the amine co-monomer is a compound of Formula I wherein R₁, R₂, R₃, and R₄ are selected from the group consisting of C₁-C₃ alkyl and C₁-C₃ alkanol. In a further embodiment, the amine co-monomer is a compound of Formula I wherein at least one of R₁, R₂, R₃, and R₄ is a C₂-C₃ alkanol group bearing at least one hydroxyl group. In a further embodiment, the amine co-monomer is an "amine-rich" alkanolamine having a ratio of the number of amine groups to the number of alkanol groups of 1:2 or more. In a further embodiment the amine co-monomer is an "amine-rich" alkanolamine having a ratio of the number of amine groups to the number of alkanol groups of 1:1 or more.

Examples of compounds of Formula I include, but are not limited to, N,N,N',N'-tetrakis(2-hydroxypropyl)ethylenediamine (THPED), N, N, N', N'-tetrakis (2-hydroxyethyl) ethylene diamine (THEED), N, N, N', N'-tetramethylethylene diamine (TEMED) (the structures of which are set forth below), enantiomers thereof, diastereoisomers thereof, and cosmetically-acceptable salts thereof. THPED is commercially available from BASF of Parsippany, New Jersey, under the tradename "Quadrol" or "Neutrol."

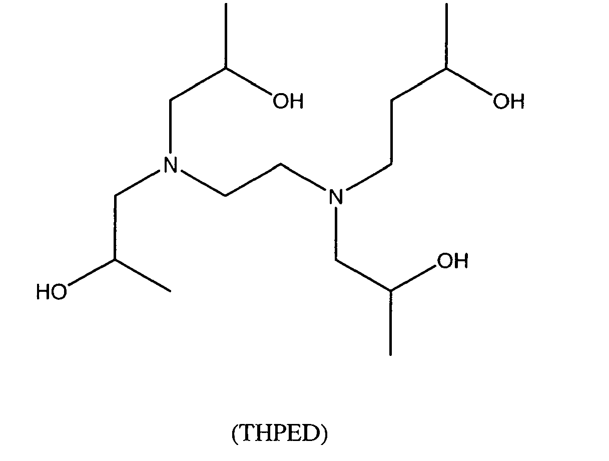

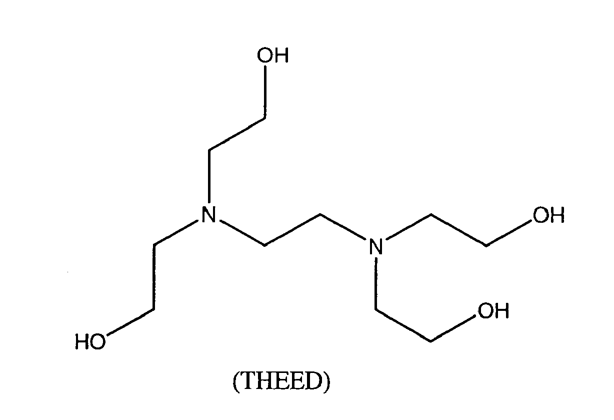

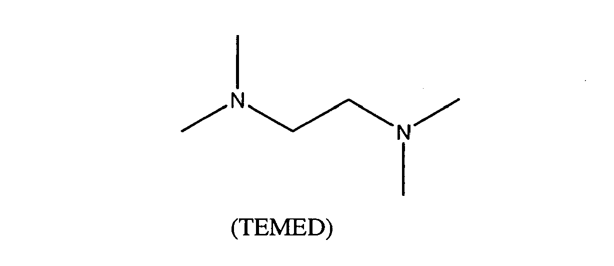

The synthesis of N,N,N',N'-tetrakis (2-hydroxypropyl) ethylenediamine from the reaction of ethylenediamine with of propylene oxide is described in U.S. Patent No. 2,697,118.

In certain other embodiments of the invention, the amine co-monomer may be a compound, in particular an alkaloamine of Formula III: 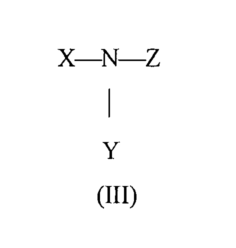 wherein each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group.

In one notable embodiment, the amine co-monomer is an alkanolamine selected from the group consisting of ethylaminoethanol, methylaminoethanol, dimethylaminoethanolamine, isopropanolamine, triethanolamine, isopropanoldimethylamine, ethylethanolamine, 2-butanolamine, choline and serine. The amine co-monomer may be an "amine-rich" alkaloamine of Formula III having a ratio of amine to alkanol of 1:2 or more. Preferably, the amine co-monomer is an "amine-rich" alkaloamine of Formula III having a ratio of amine to alkanol of 1:1 or more. More preferably, such alkanolamine is dimethylaminoethanol (DMAE).

### Additional Co-Monomer

In another embodiment, the polymer also comprises at least one repeating unit derived from one or more optional additional co-monomers, such as, for example, fatty acids such as linoleic acid, 9,10-dihydroxystearic acid, and linolenic acid; steroids such as cholesterol; and carbazole alkaloids such as dihydroxycarbazole. Such additional co-monomers can be included in the polymerization reactions carried out to make the polymer.

The melanin precursor, amine co-monomer, and additional co-monomer may be used in the form of cosmetically acceptable salts, for example non-toxic cosmetically acceptable salts, i.e., cosmetically acceptable acidic/anionic or basic/cationic salts.
Cosmetically acceptable acidic/anionic salts include those derived from acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate and triethiodide. Cosmetically acceptable basic/cationic salts include those derived from aluminum, benzathine, calcium, chloroprocaine, choline, diethanolamine, ethylenediamine, lithium, magnesium, meglumine, potassium, procaine, sodium and zinc. Other salts may also be useful, such as organic or inorganic acids including those derived from hydriodic, perchloric, sulfuric, phosphoric, propionic, glycolic, methanesulfonic, hydroxyethanesulfonic, oxalic, 2-naphthalenesulfonic, p-toluenesulfonic, cyclohexanesulfamic, saccharinic, or trifluoroacetic acid.

### Polymerization

The polymer is obtainable by reaction of the melanin precursor, amine co-monomer and, optionally, the additional co-monomer. For example, the ingredients may be mixed at room temperature, preferably less than about 50 °C, at a pH from about 7 to about 12, such as from about 9 to about 12, such as from about 9 to about 10 for a time period sufficient to react (e.g., via mixing using a conventional propeller mixer for about 1 to about 16 or more hours until the mixture turns dark/brown). The resulting polymer, which may be readily extracted from the above mixture by various means known to the art of chemical separation, has the desired color and visible/UV spectrum of other conventional, synthetic melanin polymers. The isolated polymer may be easily dissolved in water to facilitate formulation.

In one embodiment, the polymer is made at or shortly before the time of use by the consumer. In particular, the melanin precursor and amine co-monomer may be kept separated until that time, whereupon they are combined, optionally directly on the skin or hair, to form the polymer as further described below.

### Topical Compositions

In certain embodiments, the polymer is formulated into a topical composition that includes one or more additional ingredients. In one embodiment, such topical composition contain from about 0.01 % to about 10% by weight of one or more of the polymers, such as from about 0.05% to about 5% by weight of such polymer, such as from about 0.1% to about 3% by weight of such polymer. In one embodiment, the composition contains at least 0.05% by weight of such polymer, such as at least about 0.1% by weight of such polymer.

In one embodiment, the topical composition contains a safe and effective amount of (i) the polymer and (ii) a cosmetically acceptable carrier. In one embodiment, the cosmetically acceptable carrier is from about 50% to about 99.99%, by weight, of the topical composition (e.g., from about 80% to about 99%, by weight, of the composition). In a preferred embodiment of the invention, the cosmetically acceptable carrier includes or consists essentially of water.

The topical composition can be formulated as a solution. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol (200-600), polypropylene glycol (425-2025), glycerol, 1,2,4-butanetriol, sorbitol esters, 1,2,6-hexanetriol, ethanol, and mixtures thereof.

Topical compositions may be formulated as a solution comprising an emollient. Such compositions preferably contain from about 2% to about 50% of an emollient(s). As used herein, "emollients" refer to materials used for the prevention or relief of dryness, as well as for the protection of the skin or hair. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook").

Furthermore, in one embodiment, the topical composition further includes one or more additional darkening agents that do not necessarily react or chemically incorporate into the polymer of the present invention. Examples of suitable additional darkening agents include dihydroxyacetone, lawsone, erythulose, melanin, peptides, other synthetic melanin derivatives, vanillin polymers, glycerophospholipids, pigments, extracts such as but not limited to Coleus Forskoli extract, extracts from natural sources containing pigments (e.g., brown pigments from plants from the Hedychium genus or Bearberry genus or yellow, orange and red pigments from plants containing carotenoids or canthaxanthins); or synthetic chemicals such as compounds containing copper (e.g., copper salts such as CuCl₂) or synthetic carotenoids or canthaxantins. What is meant by an "extract" is a mixture of compounds isolated from a natural source (e.g., a plant).

Examples of synthetic melanin derivatives are disclosed in U.S. Patent Nos. 5,618,519, 5,384,116, and 5,227,459. Examples of soluble melanin derivatives are disclosed in 5,744,125, 5,225,435, 5,218,079, and 5,216,116. Examples of commercially available soluble melanin derivatives include Melasyn-100™ from San-Mar Laboratories, Inc. (Elmsford, NY) and MelanZe™ from Zylepsis (Ashford, Kent, United Kingdom).

These additional darkening agents will typically be present in the topical composition in an amount from about 0.001% to about 10% by weight.

In another embodiment, the topical composition may include a peptide. Examples of suitable peptides are described for example in US Patent Nos. 7,081,442, 6,797,697, and 7,025,951. Such peptides can be provided in the form of cosmetically acceptable salts. Examples of preferred salts are those derived from therapeutically acceptable organic acids, e.g., acetic, trifluoroacetic acid, palmitic, oleic, stearic, lactic, maleic, citric, malic, ascorbic, succinic, benzoic, salicylic, methanesulfonic, or pamoic acid, as well as polymeric acids such as tannic acid or carboxymethyl cellulose, and salts with inorganic acids such as the hydrohalic acids (e.g., hydrochloric acid), sulfuric acid or phosphoric acid. The amount of peptide present in the composition will depend on the peptide used. Suitable chemistry, concentrations, and synthesis of these various peptides are provided in the references noted above.

In one embodiment, the topical composition further contains another cosmetically active agent in addition to the polymer. What is meant by a "cosmetically active agent" is a compound (e.g., a synthetic compound or a compound isolated from a natural source or a natural extract) that has a cosmetic or therapeutic effect on the skin or hair, including, but not limiting to, anti-acne agents, shine control agents, antimicrobial agents, anti-inflammatory agents, sunscreens, photoprotectors, antioxidants, keratolytic agents, detergents/surfactants, moisturizers, nutrients, vitamins, energy enhancers, anti-perspiration agents, astringents, deodorants, firming agents, anti-callous agents, and agents for hair and/or skin conditioning.

In one embodiment, the agent is selected from, but not limited to, the group consisting of hydroxy acids, benzoyl peroxide, D-panthenol, octyl methoxycinnimate, titanium dioxide, octyl salicylate, homosalate, avobenzone, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q 10, peptides, amino acids such as proline, vitamins, lactobionic acid, acetyl-coenzyme A, niacin, riboflavin, thiamin, ribose, electron transporters such as NADH and FADH2, and other botanical extracts such as aloe vera, feverfew, oatmeal and derivatives and mixtures thereof. The cosmetically active agent will typically be present in the topical composition in an amount of from about 0.001 % to about 20% by weight of the composition, e.g., about 0.005% to about 10% such as about 0.01% to about 5%.

Examples of vitamins include, but are not limited to, vitamin A, vitamin Bs such as vitamin B3, vitamin B5, and vitamin B 12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants include, but are not limited to, extracts containing flavonoids and isoflavonoids and their derivatives (e.g., genistein and diadzein), extracts containing resveratrol and the like. Examples of such natural extracts include grape seed, green tea, pine bark, and propolis.

Various other materials may also be present in the topical composition. These include humectants, pH adjusters, chelating agents (e.g., EDTA), minerals, and preservatives (e.g., parabens). Examples of such agents are listed in pp. 2922-23, 2926-28, and 2892 of the ICI Handbook. In addition, the topical composition can contain conventional cosmetic adjuvants, such as dyes, opacifiers (e.g., titanium dioxide), and fragrances.

The topical composition and products containing such composition may be prepared using methodology that is well known by an artisan of ordinary skill. The topical composition may be made into a wide variety of lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable carriers including, but not limited to solutions, suspensions, emulsions such as microemulsions and nanoemulsions, gels, solids and liposomes. The following are non-limitative examples of such carriers. Other carriers can be formulated by those of ordinary skill in the art.

Lotions can be made from solutions. Lotions typically contain from about 1% to about 20% (e.g., from about 5% to about 10%) of an emollient(s) and from about 50% to about 90% (e.g., from about 60% to about 80%) of water.

Creams may also be formulated with solutions. A cream typically contains from about 5% to about 50% (e.g., from about 10% to about 20%) of an emollient(s) and from about 45% to about 85% (e.g., from about 50% to about 75%) of water.

Although it is preferred that the topical composition include water, the composition may alternatively be anhydrous or an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The topical composition may be formulated as an emulsion, containing for example from about 1% to about 10% (e.g., from about 2% to about 5%) of an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The topical composition can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The topical composition can also be formulated into a solid formulation (e.g., a wax-based stick, soap bar composition, powder, or a wipe containing powder).

It is also contemplated that the polymer of the present invention may be formed into a liposome such as a unilamellar, multilamellar, and paucilamellar liposome. Such liposomes can be prepared by combining the polymer and optionally other ingredients and water. The liposome may also be incorporated into one of the above cosmetically acceptable carriers (e.g., a gel or an oil-in-water emulsion).

Micelle formulations are also useful topical compositions of the present inventions. Such micelle compositions are disclosed in the U.S. Patent No. 6,284,234.

Other encapsulation technologies are also useful in the topical composition, such as porous beads as those described in U.S. Patent Nos. 4,690,825 and 5,145,675.

The topical composition may contain, in addition to the aforementioned components, a wide variety of additional oil-soluble materials and/or water-soluble materials conventionally used in compositions for use on skin and hair, at their art-established levels.

### Methods of Use

The polymer may be applied topically to human skin or hair in the form of a topical composition as described above. In an alternative embodiment, the polymer may be applied to the skin directly (in an "unformulated" or "neat" state) for a time period sufficient to allow the polymer to absorb into the skin. In another embodiment, the polymer is formed, optionally on the skin or hair, at or shortly before use by the consumer.

Products made using the invention may be packaged as known in the art. In one embodiment, the package is a container such as a plastic, metal, or glass tube or jar containing the composition. Such products may further contain additional packaging such as a plastic or cardboard box for storing such container. In one embodiment, instructions directing the user to apply the polymer or topical composition to the skin or hair to darken the skin (e.g., to tan the skin), even skin tone (e.g., to darken light areas of the skin or to correct or conceal the mottled hyperpigmentation), or darken the hair (e.g., to darken light brown, blonde, gray or white hairs) are included. Such instructions may be printed on the container, label insert, or on any additional packaging.

In one embodiment, the melanin precursor and the amine co-monomer are separated from one another (such as by a barrier or the use of separate containers) until the point of use, at which time they are applied to the skin and allowed to react thereon. Accordingly, one method of darkening the skin comprises 1) applying to the skin a first composition comprising the melanin precursor; and 2) applying to the skin a second composition comprising the amine co-monomer. The melanin precursor and the amine co-monomer are then allowed polymerize on the skin, thereby darkening the skin. Either or both of the melanin precursor and the amine co-monomer may be formulated with various ingredients as described herein. In one embodiment, at least one of the melanin precursor and the amine co-monomer are formulated so as to appear light-colored, e.g., white such as in a milky or creamy lotion base.

Thus, according to another aspect of the invention, a product useful for darkening the skin includes a first composition in a first container, wherein the first composition comprises the melanin precursor; and a second composition in a second container separated from said first container comprising the amine co-monomer. The melanin precursor and the amine co-monomer may be separated by a barrier, e.g., a plastic, that is substantially impermeable to both the melanin precursor and the amine co-monomer. The barrier may be a wall such as a plastic wall that is typically employed in "dual chamber" packaging for applying toothpaste or cosmetics; or a dual chamber spray bottle such as those used for cleaning solutions, etc. In this scenario, the melanin precursor and the amine co-monomer come into contact on the skin, and react to form the polymer.

The following non-limiting example further illustrates the invention.

### Example I : Synthesis of Polymers and Evaluation of Color and Absorbance

The following preparations shown in Table 1 were made: comparative examples C1, C2, C8, C13, C18 and C19 and inventive examples E3, E4, E5, E6, E7, E9, E10, E11, E12, E14, E15, E16, and E17. The preparations were made by mixing "Melanin precursor 1" with "Melanin precursor 2" (when present) using a standard propeller mixer. In each case, the concentration of total melanin precursor (Melanin precursor 1 + Melanin precursor 2) was 0.05%. For those examples in which " Melanin precursor 2" was used, the weight ratio of Melanin precursor 1 to Melanin precursor 2 was 1:1.8. In the inventive examples, an amine-co-monomer was mixed with the melanin precursors at ambient temperature. For these examples the weight percentage of amine-co-monomer in the total mixture is indicated in Table 1. The balance of each mixture was a vehicle consisting of water. The pH of each mixture was measured after mixing immediately and was adjusted upward (when no amine co-monomer was used in the example) using NaOH or downward using HCl (typically when an amine co-monomer was used in the example).

The color of each example was evaluated by placing a sample of about 200 microlieters of the mixture in a standard assay plate (of a 96-well plate). This evaluation was performed after 1 hour and again after 16 hours of mixing. Absorbance at 600nm was evaluated after 90 minutes of mixing using a conventional UV-VIS spectrophotometer (SpectraMax Plus³⁸⁴, Molecular Device, Sunnyvale, CA). The results are shown in Table 1.

**Table 1**

| Example | Melanin precursor 1 | Melanin precursor 2 | Amine Co-monomer | Amine Concen tration (%) | pH | Color at 1 hr | Color at 16 hr | A_{600 nm} |
|---|---|---|---|---|---|---|---|---|
| C1 | Aloin | | | 0 | 7.2 | light brown | Light-medium brown | 0.15 |
| C2 | Aloin | | | 0 | 12.1 | Light-medium brown | Medium organge-brown | 0.15 |
| E4 | Aloin | | THPED | 0.5% | 9.7 | Medium brown | Medium brown | 0.2 |
| E5 | Aloin | | THPED | 0.5% | 7.2 | light brown | Light-medium brown | 0.15 |
| E6 | Aloin | | DMAE | 0.5% | 10.2 | Medium-high brown | Medium brown | 0.35 |
| E7 | Aloin | | DMAE | 0.5% | 7.2 | Light brown | medium brown | 0.15 |
| C8 | Aloin | Vanillin | | 0 | 7.2 | Very light brown | Very light brown | 0.03 |
| E9 | Aloin | Vanillin | THPED | 0.5% | 9.7 | Light brown | Light brown | 0.13 |
| E10 | Aloin | Vanillin | THPED | 0.5% | 7.2 | Very light brown | Very light brown | 0.1 |
| Ell | Aloin | Vanillin | DMAE | 0.5% | 10.2 | Light-medium brown | Light-medium brown | 0.2 |
| E12 | Aloin | Vanillin | DMAE | 0.5% | 7.2 | Very light brown | Light dark-brown | 0.15 |
| C13 | Aloin | DOPA | | 0 | 7.2 | Very light brown | Light blackish brown | 0.1 |
| E14 | Aloin | DOPA | THPED | 0.5% | 9.7 | Light-medium brown | Blackish brown | 0.4 |
| E15 | Aloin | DOPA | THPED | 0.5% | 7.2 | Very light brown | Light blackish brown | 0.1 |
| E16 | Aloin | DOPA | DMAE | 0.5% | 10.2 | Medium-high brown | Medium-high brown | 0.5 |
| E17 | Aloin | DOPA | DMAE | 0.5% | 7.2 | Very light brown | Light blackish brown | 0.1 |
| C18 | Aloin | DOPA | TEA | 0.75% | 9.1 | Greenish light brown | Blackish brown (at 4hr) | 0.4 (at 2hr) |
| C19 | 0.05% MELASYN | | | | | Medium-high brown | | 0.6 |

As indicated in Table 1, the comparative examples either provided low absorbance (0.15 or less), indicating both low degree of polymerization and low ability to induce darkening of the skin, or, in the case of C 18, provided a green or black color that was not aesthetically appealing. In contrast, the inventive examples provide higher absorbance, particularly when the pH was adjusted from 7.2 to above 9. Comparative example C19 was Melasyn-100™, a skin darkening compound commercially available from San-Mar Laboratories, Inc. (Elmsford, NY). While MELASYN-100™ provided acceptable color and absorbance, it is a synthetic melanin that is produced using a process utilizing harsh oxidative chemicals at relative high temperatures.

### Example II : Synthesis of Polymers and Evaluation of Color and Absorbance

The following preparations shown in Table 2 were made: comparative examples C20-C22 and inventive examples E23-E30. The preparations were made as described in Example 1. The color evaluation was performed after 2 hours and again after 4 hours of mixing.

**Table 2**

| Example | Melanin precursor 1 | Melanin precursor 2 | Amine Co-monomer | Amine Concentr ation (%) | pH | Color at 2 hr | Color at 4 hr |
|---|---|---|---|---|---|---|---|
| C20 | Aloin | DOPA | | 0 | 7.1 | Very light brown | Light blackish brown |
| C21 | Aloin | DOPA | | 0 | 9.1 | Light brown | Medium blackish brown |
| C22 | Aloin | DOPA | | 0 | 10.3 | Medium brown | Blackish brown |
| E23 | Aloin | DOPA | THPED | 0.5% | 7.1 | Very light brown | light brown |
| E24 | Aloin | DOPA | THPED | 0.5% | 9.1 | Medium-high brown | Blackish brown |
| E25 | Aloin | DOPA | THPED | 0.5% | 10.3 | Medium-high brown | Blackish brown |
| E26 | Aloin | DOPA | THPED | 0.5% | 12.1 | Orange brown | Medium-dark brown |
| E27 | Aloin | DOPA | DMAE | 0.5% | 7.1 | Very light brown | Light brown |
| E28 | Aloin | DOPA | DMAE | 0.5% | 9.1 | Medium-dark brown | Dark brown |
| E29 | Aloin | DOPA | DMAE | 0.5% | 10.3 | Medium- brown | Medium-dark brown |
| E30 | Aloin | DOPA | DMAE | 0.5% | 12.1 | Orange-brown | Medium brown |
| C19 | 0.05% melasyn | | | | | Medium brown | |

The data in Table 2 suggests that intensity of color can be modulated with pH, and generally intensity can be enhanced by increasing pH from about 7 to about 12. It also suggests that DMAE as an amine co-monomer (an alkanolamine with a ratio of amine to alkanol of 1) is somewhat better than THPED (an alkanolamine with a ratio of amine to alkanol of 2) for providing medium brown tones particularly at moderate pH. However, either class of amine may be suitable, particularly if a range of colors is desired.

### Example III : Application of Polymers and Comparative Examples To Human Skin

Two mixtures were prepared: A: 2% Quadrol in a vehicle of ethanol:propylene glycol (70:30 v/v); and B: 0.5% aloin in the same vehicle. A Caucasian individual with a light skin color was treated, on three different sites (a, b and c) of her arm, with the following:
site (a): 10 microliters of A,
site (b): 10 microliters of B, and
site (c): 5+5 microliters of A and B mixed directly on her arm.

Immediately on the drying of the material, visual observations showed no change of color on site (a), a minimal/barely visible change of skin color on site (b), and a nice, tan-like color on site (c). The tan-like color for site (c) remained on the skin during the day. Upon washing the sites, the intensity of the tanned color was reduced, but it was still visible.

Additional applications of each test material were made to the same, corresponding skin sites (the next morning and following evening), with routine washing. The pleasant tan-like color remained for site (c) whereas site (a) showed no change in color, and site (b) remained barely visible.

This example demonstrates that the described invention can be used to create tanned-like appearance on a human skin, immediately after topical application, with a lasting effect.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A polymer comprising (i) at least one repeating unit derived from a melanin precursor selected from the group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin, and mixtures thereof; and (ii) at least one repeating unit derived from an amine co-monomer having a formula selected from the group consisting of: 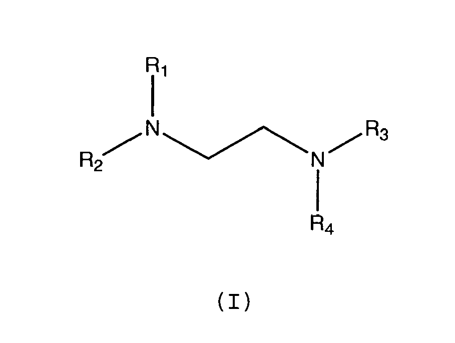 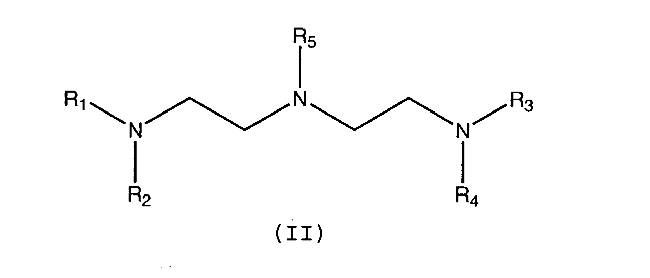 and 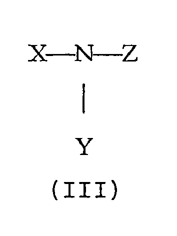 wherein each of R₁, R₂, R₃, R₄, and R₅ are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof.

2. The polymer of claim 1, wherein said amine co-monomer is a compound of Formula I wherein each of R₁, R₂, R₃, and R₄ are selected from the group consisting of C₁-C₃ alkyl and C₁-C₃ alkanol.

3. The polymer of claim 1, wherein said amine co-monomer is a compound of the formula: 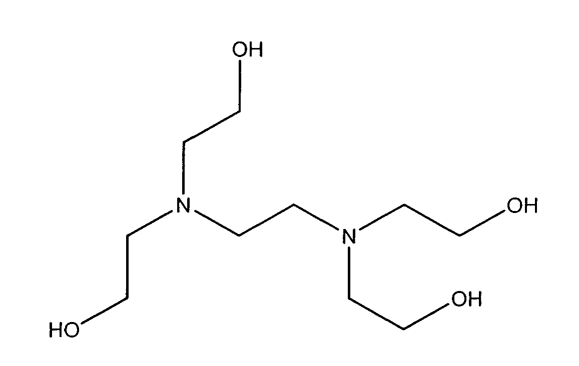 or a cosmetically acceptable salt thereof.

4. The polymer of claim 1, wherein said amine co-monomer is a compound of the formula 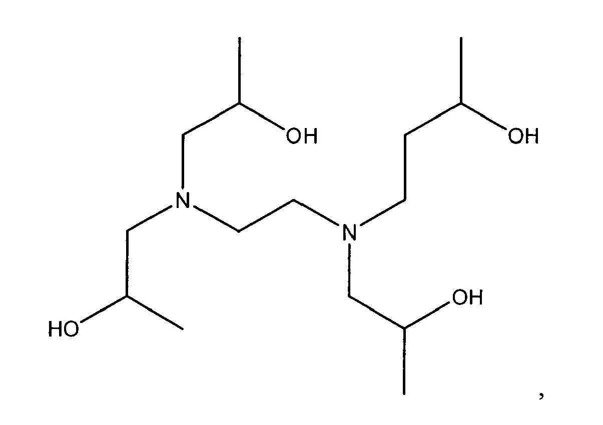 an enantiomer thereof, a diastereoisomer thereof, or a cosmetically acceptable salt thereof.

5. The polymer of claim 1, wherein said amine co-monomer is an alkanolamine selected from the group consisting of ethylaminoethanol, methylaminoethanol, dimethylaminoethanol-amine, isopropanolamine, triethanolamine, isopropanoldimethylamine, ethylethanol-amine, 2-butanolamine, choline, serine, and combinations thereof.

6. The polymer of claim 1 or claim 5, wherein said amine co-monomer is an alkanolamine having an amine to alkanol ratio of at least 1:2, preferably at least 1:1.

7. The polymer of any preceding claim, wherein said melanin precursor comprises aloin, dihydroxyphenylalanine, vanillin, and combinations thereof.

8. A topical composition comprising the polymer of any preceding claim and a cosmetically acceptable carrier.

9. A method of darkening the skin, said method comprising applying to said skin a polymer of any one of claims 1 to 7.

10. A polymer of any one of claims 1 to 7 for use for darkening the skin.

11. Use of the polymer of any one of claims 1 to 8 for the manufacture of a medicament for darkening the skin.

12. A method of darkening the skin, said method comprising:
applying to said skin a first composition comprising a melanin precursor selected from the group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin, and mixtures thereof;
applying to said skin a second composition comprising an amine co-monomer having a formula selected from the group consisting of: 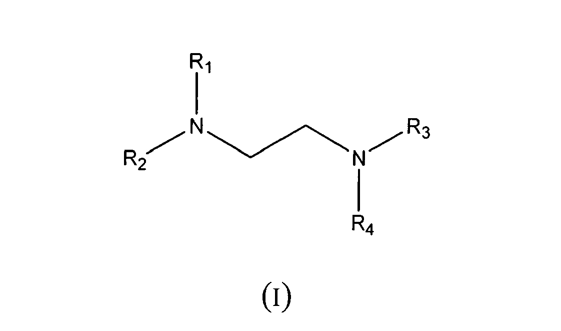 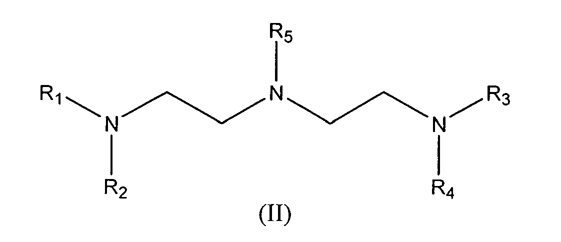 and 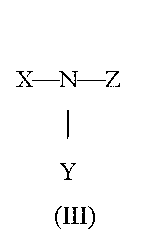
wherein each of R₁, R₂, R₃, R₄, and R₅ are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof; and
allowing said melanin precursor and said amine co-monomer to polymerize on said skin.

13. The method of claim 12, wherein said applying steps are performed at a temperature no higher than about 50°C.

14. A product comprising:
a first composition in a first container, wherein said first composition comprises a melanin precursor selected from the group consisting of 3-aminotyrosine, dihydroxy acetone, 3,4-dihydroxybenzoic acid, 3-amino,4-hydroxybenzoic acid, aloin, emodin, alizarin, tyrosine, dihydroxyphenylalanine, 4,5-dihydroxynaphthalene-2-sulfonic acid, 3-nitrotyrosine, 3-dimethylamino phenol, p-aminobenzoic acid, vanillin and mixtures thereof; and
a second composition in a second container separated from said first container, wherein said second composition comprises an amine co-monomer having a formula selected from the group consisting of: 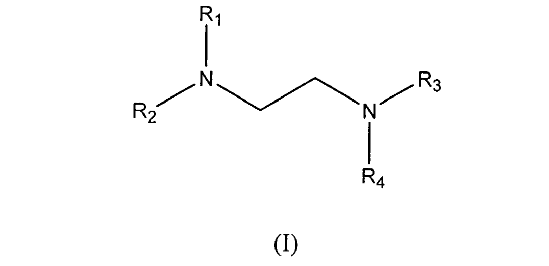 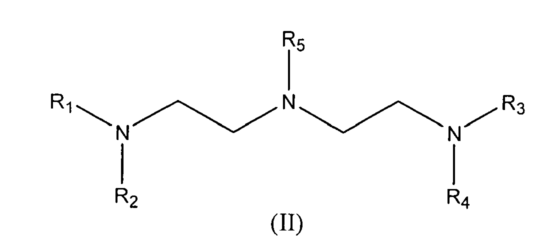 and 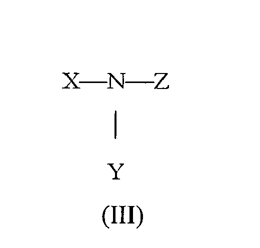
wherein each of R₁, R₂, R₃, R₄, and R₅ are selected from the group consisting of hydrogen, C₁-C₆ alkyl, C₁-C₆ hydroxyalkyl, each of X, Y and Z are selected from the group consisting of hydrogen, C₁-C₃ alkyl group, C₂-C₄ alkanol group, and at least one of X, Y or Z is a C₂-C₄ alkanol group bearing at least one hydroxyl group and optionally at least one carboxyl group, or cosmetically acceptable salts thereof.

15. The product of claim 14, wherein said first and second compositions are separated from one another by a substantially impermeable barrier.

16. A product of claim 14 or claim 15 for use for darkening the skin wherein the first composition comprising the melanin precursor is applied to the skin and the second composition, comprising the amine co-monomer is applied to the skin such that the melanin precursor and the amine co-monomer polymerize on the skin.
